# EUROPEAN PATENT APPLICATION

(11) **EP 4 420 670 A1**
(43) Date of publication of application: **28.08.2024**
(21) Application number: 23382179.2
(22) Date of filing: 27.02.2023
(51) Int. Cl.: A61K 38/00, A61K 47/64, C07K 19/00, A61K 47/62, C07K 7/00, G01N 33/58, G01N 33/68, G01N 33/50, A61K 47/69, C07K 14/00, C12N 9/36, G01N 33/569

(54) **NON-COVALENT BINDING PEPTIDE AND CARRIER COMPLEX COMPRISING IT**

(71) Applicant: Consejo Superior De Investigaciones Científicas, 28006 Madrid (ES)
(72) Inventor: Pérez Martínez, Gaspar, Valencia (ES); Bäuerl, Christine, Valencia (ES)
(74) Representative: Pons

(57) **Abstract**

The invention relates to a positively charged peptide capable of non-covalently interact with negatively charged extracellular vesicles or nanoparticles. Said peptide is a fragment of the bacterial protein P40 and shows better interaction capabilities that the entire bacterial protein P40. The peptide of the invention can also be linked to a targeting element, such as targeting peptide, DNA aptamer, RNA aptamer, a protein, an antibody or a fragment thereof that binds to a target molecule, therefore it can be used for the targeted delivery of extracellular vesicles or nanoparticles further comprising a bioactive agent to a target cell, tissue or particle like a virus, that comprise the target molecule. Therefore, the present invention also relates to a carrier complex comprising the peptide of the invention non covalently bound to an extracellular vesicle or nanoparticle comprising a bioactive agent and its uses.

## Description

The invention relates to a peptide capable of non-covalently interacting with negatively charged extracellular vesicles or nanoparticles. The invention also relates to said peptide further comprising a targeting element and its use for the targeted delivery of nanoparticles or extracellular vesicles comprising a bioactive agent to a target particle such a virus, bacteria, a target cell or tissue. Thus, the present invention is within the biotechnology and biomedicine field.

### BACKGROUND ART

When administering a bioactive agent, such as a drug, different challenges may arise related to the toxicity associated with an effective concentration. Moreover, *in vivo* degradation of bioactive agents can occur, as well as the absorption/retention of the agent by non-targeted systems. So, one of the new trends in biotechnology and biomedicine field is the development of new strategies that improve the delivery of a bioactive agent to a specific cell or tissue, where they exert an effect such as therapeutic or toxic effect.

The use of targeted extracellular vesicles (EVs), as well as artificial nanoparticles such as liposomes, micelles or mesoporous silica nanoparticles that comprise cargo molecules, such as a therapeutic agent, have been used to solve these issues. Targeted extracellular vesicles or nanoparticles are usually modified by the incorporation of a moiety with a specific affinity to a target molecule, the target molecule being present in the desired cells or tissue where the cargo molecule is intended to be delivered.

EVs, such as exosomes and microvesicles, are secreted by cells of virtually all living kingdoms that perform cell to cell communication functions. EVs carry cargoes from donor cells in the form of proteins, lipids and nucleic acids that are taken up by the recipient cells. Therefore, due to their natural functions, EVs can be used for the delivery of bioactive agents (cargoes) to a target. Compared to other nanomaterials, EVs show the advantages that are totally innocuous, biocompatible, have better delivery efficiency and lower immunogenicity, although there are some disadvantages like lack of targeting capability, low production yields and on-demand drug release. So, the use of targeted EVs for the delivery of bioactive agents has been extensively studied in the recent years (Armstrong, J. P., Holme, M. N., & Stevens, M. M. 2017. ACS nano, 11(1), 69-83).

Both covalent and non-covalent modification approaches are used to modify the surface of EVs with targeting elements. The covalent modification approaches are based on the binding of a targeting element to the surface of EVs by means of covalent bonds (Jia, G., et al., (2018). Biomaterials, 178, 302-316). However, covalent modification approaches involve chemical modification of EVs surface and might change the physicochemical properties of EV membrane.

On the other hand, non-covalent approaches take advantage of the natural features of the EV. For example, a targeting element can be fused to an amphiphilic moiety that can assemble itself into the phospholipid bilayer of EVs by means of hydrophobic interactions (Wang J, et al., Advanced Functional Materials 2018, 28(18):1707360). Interestingly, EVs from different organisms have some common physicochemical features, such their small size, but notably they have generally negative charges. For example, Gram-positive and Gram-negative microorganisms contain membrane anchored negatively charged molecules, respectively, lipoteichoic acid and lipopolysacharides, that have an essential structural role in the cell wall and that confer the overall negative charge to the bacterial surface (Kim, J. et al., (2015, April). Seminars in cell & developmental biology (Vol. 40, pp. 97-104)). EVs from higher organisms including human (Midekessa, Get al., (2020) Acs Omega, 5(27), 16701-16710) and plant EVs (Nemidkanam, V., & Chaichanawongsaroj, N. (2022). Plos one, 17(1), e0262884) also possess negatively charged surface. Therefore, negative charge feature could be used for non-covalent modification strategies of EVs with targeting elements.

In summary, the provision of new approaches based on non-covalent modifications is considered as a promising alternative in the development of targeted EVs for the delivery of bioactive agents to a target such as a pathogen like a virus, bacteria, a target cell or tissue.

### DESCRIPTION OF THE INVENTION

The inventors have surprisingly found that peptide fragments P40N1 (SEQ ID NO: 1), and P40N2 (SEQ ID NO: 2) of the bacterial protein P40 (NCBI Reference Sequence: CAQ65155.1, SEQ ID NO: 3), are positively charged peptides that can establish non-covalent interactions, such as electrostatic interactions, with negatively charged molecules or particles, such as heparin, showing higher affinity than the entire bacterial protein P40 (Table 1). The bacterial protein P40 is a muramidase secreted by bacteria of the *Lactobacillus* genus and said present in the cell surface. The protein P40 belongs to a large family of secreted cell wall proteins that contain a carboxy(C)-terminal histidinedependent amino hydrolase/peptidase (CHAP) domain, a characteristic domain of cellwall hydrolase activity (Bäuerl, C., et al., 2010. Journal of Molecular Microbiology and Biotechnology. 19(4), 231-241).
peptide P40N1 SEQ ID NO: 1: 
DTSDSIASNKSETNALLKQIEDANTEVINLNKQIDTKNGQISDATAKISATDAKISSLSGE
IVAAQKNVTARKNNLKDQLISLQKKAGNSVSGNVYIDFVLNSQNLSDLIARTMTVGKLS
QASKDALDAVTVAKDKLAGLKTEQENARQTLVSTKASLETQKSQLVSLQKDANDKQD
ALNKQ
peptide P40N2 SEQ ID NO: 2: 
DTSDSIASNKSETNALLKQIEDANTEVINLNKQIDTKNGQISDATAKISATDAKISSLSGE
IVAAQKNVTARKNNLKDQLISLQKKAGNSVSGNVYIDFVLNSQNLSDLIARTMTVGKLS
QASKDALDAVTVAKDKLAGLKTEQENARQTLVSTKASLETQKSQLVSLQKDANDKQD
ALNKQIADHKDELVALQSQFAQEQSEAAAATQAALKTVAASTASSSTTSSSSSATSTS
TIANNTSSSSNNSSSIINNNTTSGSGSHADYSSSGN
Bacterial protein P40 [*Lacticaseibacillus paracasei* BL23], NCBI reference sequence CAQ65155.1, SEQ ID NO: 3 .

MKFNKVMITLVAAVTLAGSASAVTPVFADTSDSIASNKSETNALLKQIEDANTEVINLNK
QIDTKNGQISDATAKISATDAKISSLSGEIVAAQKNVTARKNNLKDQLISLQKKAGNSVS
GNVYIDFVLNSQNLSDLIARTMTVGKLSQASKDALDAVTVAKDKLAGLKTEQENARQT
LVSTKASLETQKSQLVSLQKDANDKQDALNKQIADHKDELVALQSQFAQEQSEAAAA

TQAALKTVAASTASSSTTSSSSSATSTSTIANNTSSSSNNSSSIINNNTTSGSGSHADY
SSSGNTYPWGQCTWYVKSVASWAGNGWGNGAQWGSSAAAAGFTVNHTPAAGSIIV
FAAGQSVGGQWTADGSYGHVAYVQSVSGDSVTISQGGMGFSSPTGPNTQTISGASS
YVVYIHR

Other NCBI references of the bacterial protein P40 include WP_014951485.1, WP_003572828.1 or WP_260340977.1.

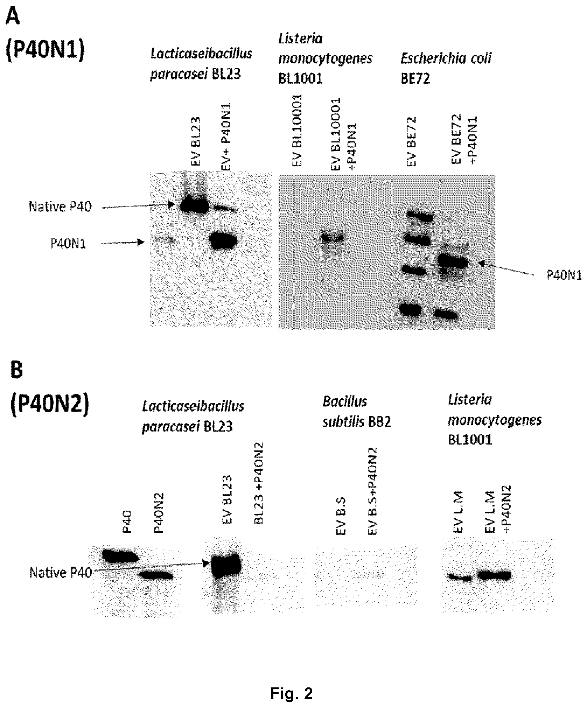

Moreover, the inventors tested the binding capacity of fragments of bacterial P40 (Peptides P40N1 and P40N2) for EVs from different Gram-positive bacteria and Gram-negative bacteria. Peptides P40N1 and P40N2 surprisingly displaced native P40 from the surface of EVs of *Lactobacillus* bacteria, likely due to its strong interaction with negatively charged molecules on the surface of EVs. In addition to this, the inventors also found that the tested fragments were able to bind to the surface of EVs from other bacteria belonging to the genus *Bacillus, Listeria* and *Escherichia* (Figure 2).

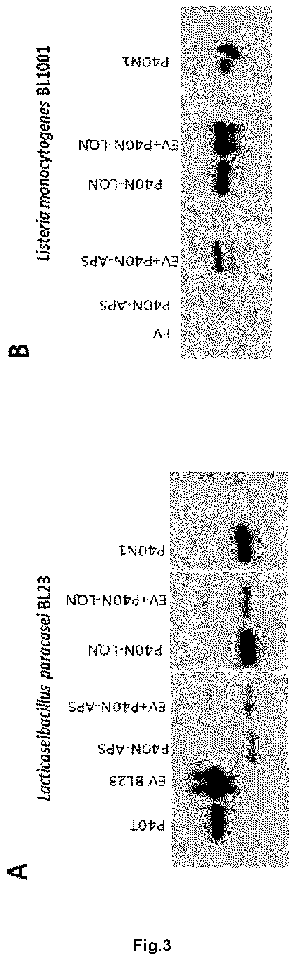

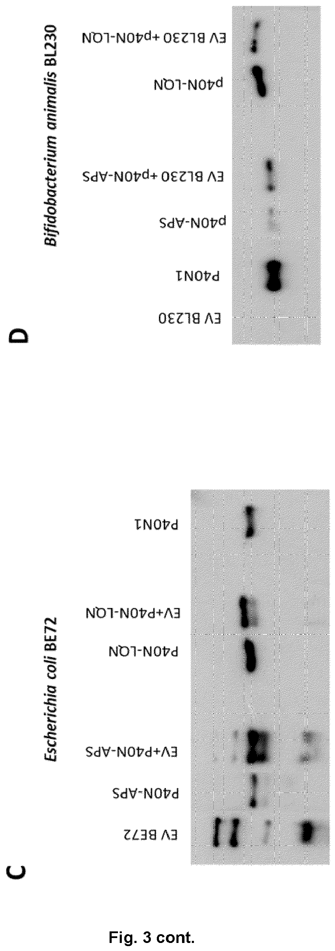

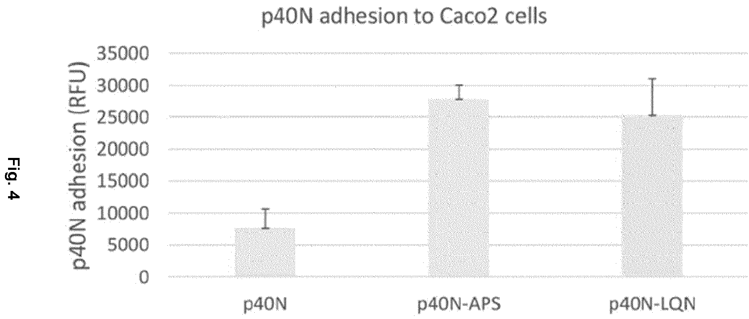

In order to provide target recognition properties to these peptides, the inventors engineered fusion proteins comprising P40N1 linked to cancer stem cells-recognizing heptapeptides. The resulting constructs maintained the ability to bind to bacterial EVs (Figure 3). Adhesion experiments to Caco-2 cell line demonstrated that fusion proteins comprising P40N1 and targeting peptides were able to bind adenocarcinoma cells (Figure 4).

Based on these results, it is proposed that the fragments of bacterial P40 can be used for the development of a universal molecular bridge further comprising a targeting element, that allows fast and technically simple targeting and delivery of EV (prokaryotic or eukaryotic), or artificial negatively charged nanoparticles, loaded with a broad range of cargoes to specific cells, tissues, organs or pathogen targets like viruses.

Sequences of P40N1 (SEQ ID NO:1) and P40N2 (SEQ ID NO:2) are found in the amino terminal domain of bacterial protein P40. They belong to the COG3883 domain family (Database of Clusters of Orthologous Genes, NCBI), the only member of the superfamily cl25603, which is found in some surface proteins and antigens of different Gram-positive bacteria (Firmicutes), such as *Bacillus subtilis, Bacillus halodurans, Clostridium acetobutyricum, Lactococcus lactis, Streptococcus pneumoniae, Streptococcus pyogenes* and *Listeria innocua,* among others. Domains are protein regions, COG stands for Clusters of Orthologous Groups of protein sequences (COG), and COG domain families are groups of sequences that are "more similar to each other than they are to any other proteins from the same genomes" (Tatusov et al., 2000. Nucl Acids Res, 28, 33-36).

Thus, one aspect of the present invention refers to a peptide, hereinafter the "peptide of the invention" comprising an amino acid sequence with at least 80% sequence identity to the amino acid sequence SEQ ID NO: 1, wherein the length of the peptide is less than 300 amino acids.

In the context of the present invention, the term "sequence identity" or "identity" is understood to mean the degree of similarity between two nucleotides or amino acid sequences obtained by aligning the two sequences. Depending on the number of common residues between the aligned sequences, a different degree of identity, expressed as a percentage, will be obtained. The degree of identity between two amino acid sequences may be determined by conventional methods, for example, by standard sequence alignment algorithms known in the state of the art, such as, for example, BLAST [Altschul S.F. et al. Basic local alignment search tool. J Mol Biol. 1990 Oct 5; 215(3): 403-10]. The BLAST programs, for example, BLASTN, BLASTX, and BLASTX, BLASTP and TBLASTN, are in the public domain at The National Center for Biotechonology Information (NCBI) website. The skilled person in the art understands that mutations in the nucleotide sequence of genes that lead to conservative amino acid substitutions at non-critical positions for the functionality of the protein are evolutionarily neutral mutations which do not affect its global structure or its functionality. Thus, amino acids or nucleotides sequences with identities of at least 80% can be considered to retain the same properties as the sequence to which they refer.

In a preferred embodiment, the peptide of the invention comprises an amino acid sequence with at least 85%, 90%, 91%, 92%, 93%, 94%, 95%, 96%, 97%, 98% or 99% of identity with the amino acid sequence SEQ ID NO: 1.

Peptides P40N1 y P40N2 comprise positively charged amino acids that enable their interaction with negatively charged EVs or nanoparticles by means of electrostatic interactions. The amino acid sequences of peptides P40N1 and P40N2 comprise at least 1 out of 10 positively charged amino acids like K (Lysine, Lys) and R (Arginine, Arg).

In a preferred embodiment of the peptide of the invention, at least 1 out of 10 amino acids of the amino acid sequence is a positively charged amino acid.

The expression "positively charged amino acid", as used herein, refers to amino acids whose side chains are positively charged. Amino acids may be positively charged at physiological pH conditions (pH ~ 7). Positively charged amino acids include K (Lysine, Lys) and R (Arginine, Arg).

In a preferred embodiment, the peptide of the invention comprises the amino acid sequence SEQ ID NO: 1 or SEQ ID NO: 2.

In another preferred embodiment, the peptide of the invention consists of the amino acid sequence SEQ ID NO: 1 or SEQ ID NO: 2.

As understands the skilled person in the art, the peptide of the invention can be linked to target-recognizing element (or targeting element).

So, in another preferred embodiment, the peptide of the invention is covalently linked to a targeting element, wherein the targeting element is covalently linked to the N-terminal end, C-terminal end, or any other amino (-NH₂), carboxy (-COOH), sulphide (S²-) or reactive groups of the peptide of the invention.

The terms "covalently" or "covalent bond", used interchangeably, refer to a chemical bond that involves the sharing of electrons to form electron pairs between atoms, an example of covalent bond is the peptide bond.

In the context of the present invention, the terms "targeting element" or "targeting moiety", used interchangeably, refer to a molecule or macromolecule that specifically binds or interact with a target molecule. The "target molecule" refers to a molecule that is specifically bound by a targeting element, wherein the target molecule may be a peptide, protein, a carbohydrate, a lipid or a nucleic acid. The target molecule is the cognate binding partner of the targeting element.

The term "specifically binds", when referring to the interaction of a targeting element or moiety and its cognate binding partner, refers to a binding reaction which is determinative of the presence of the targeting element and its cognate molecule in the presence of a heterogeneous population of molecules (e. g., proteins, peptides, lipids, carbohydrates or nucleic acids).

The target molecule may be part of a cell, tissue, or particle such a virus. Therefore, the targeting element interacts, binds or directs the peptide of the invention to a cell, tissue, organ or particle such as virus having the targeting molecule to which a specific targeting element binds.

In another preferred embodiment, the targeting element linked to the peptide of the invention is selected from the list consisting of: a RNA aptamer, a DNA aptamer, a polysaccharide, a targeting peptide, a protein, an antibody and a fragment thereof.

The term "RNA aptamer" refers to a short single stranded RNA (ribonucleic acid) molecule that can specifically bind to a target molecule, wherein the length of the RNA aptamer is from 10 to 100 nucleotides, preferably from 20-80 nucleotides.

The term "DNA aptamer" refers to a short single stranded DNA (deoxyribonucleic acid) molecule that can specifically to a target molecule, wherein the length of the RNA aptamer is from 10 to 100 nucleotides, preferably from 20-80 nucleotides.

The term "polysaccharide" refers to a polymeric carbohydrate composed of monosaccharide units bound by glycosidic linkages.

The terms "targeting peptide" or "targeting polypeptide" refer to a peptide having a sequence length ranging from 3 to 100 amino acids, directed to a target molecule. As example, the targeting peptide may be a peptide that specifically binds to a protein on the surface of a target cell such as a tumour cell.

The term "protein" being a targeting element refers to a protein having a sequence length ranging from 100 to 600 amino acids conformed by one or more domains, wherein said protein is directed to a target molecule as defined above. However, a protein being a targeting element may also be a protein with more than 600 amino acids. For example, the protein being a targeting element may be albumin that would bind to cells expressing albumin receptors on their surface.

The term "antibody" or "antibodies", as used herein, refers to immunoglobulin molecules and immunologically active portions of immunoglobulin molecules (i.e., molecules that contain an antigen binding site that immuno-specifically binds an antigen, specifically the antigen can be a target molecule as defined above). The term also refers to antibodies comprised of two immunoglobulin heavy chains and two immunoglobulin light chains as well as a variety of forms including full length antibodies and portions or fragments thereof; including, for example, an immunoglobulin molecule, a monoclonal antibody, a chimeric antibody, a CDR-grafted antibody, a humanized antibody, a Fab, a Fab', a F(ab')2, a Fv, a disulfide linked Fv, a scFv, a single domain antibody (dAb), a diabody, a multispecific antibody, a dual specific antibody, an anti-idiotypic antibody, a bispecific antibody, a functionally active epitope-binding fragment thereof, bifunctional hybrid antibodies. The antibody may be of any type (e.g., IgG, IgA, IgM, IgE or IgD). Preferably, the antibody is IgG. An antibody may be non-human (e.g., from mouse, goat, or any other animal), fully human, humanized, or chimeric. As illustrative examples, the antibody (or a fragment thereof) linked to the peptide of the invention include, without limitation: an antibody that binds to Her2 (e.g Trastuzumab); an antibody that binds to CD20 (e.g Rituximab); or an antibody that binds to a VEGF (e.g bevacizumab).

Particularly, the targeting element is capable of specifically binding, interacting, localizing a target cell, a tissue, a bacteria or virus, particularly recognizing a cell marker, a tissue marker or a molecule, such as protein, on the surface of the target cell, target tissue, target bacteria or virus.

The term "cell marker" refers to a molecule present in a specific type of cell, thus it allows to identify and classify said cell differentiating it from other cells that do not present it. The cell can be a eukaryotic cell or a prokaryotic cell. The eukaryotic cell may be a cancer cell. The prokaryotic cell may be a bacterium cell, wherein the bacterium may be a pathogenic bacterium.

The expression "tissue marker" refers to a molecule present on a specific cell population being part of a tissue in a complex organism, such as connective tissue, muscular tissue, epithelial tissue or nervous tissue or a pathological tissue such as a tumoral tissue. Examples cell or tissue markers are CD19, CD20, VEGFR, Disialoganglioside GD2 or ganglioside GM2. Preferably, the cell or tissue marker to which the targeting element binds is a receptor. The term "receptor" refers to a protein that is present inside or on the surface of a cell that binds to a substance or ligand, for example the ligand is a peptide or a protein, and when a ligand binds to its receptor, the receptor can change conformation, transmitting a signal into the cell.

As the skilled person in the art knows, the targeting element may be a targeting peptide, such as, but not limited to: a peptide RGD (arginine-glycine-aspartate), that specifically binds to integrin receptors; a peptide comprising, preferably consisting of, the sequence SEQ ID NO: 4: LQNAPRS, that specifically binds to CD133; a peptide comprising, preferably consisting of, the amino acid sequence SEQ ID NO: 5: APSPMIW, that specifically binds to CD133; a peptide comprising, preferably consisting of, the amino acid sequence SEQ ID NO: 6: QMAQWPP, that specifically binds to CXCR7; a peptide comprising, preferably consisting of, the amino acid sequence SEQ ID NO: 7: APLPRPG to that specifically binds to CXCR7; or a peptide comprising, preferably consisting of, the amino acid sequence SEQ ID NO: 8 ATWLPPR that specifically binds to VEGF receptor.

In another embodiment, the targeting element linked to the peptide of the invention is a targeting peptide comprising, preferably consisting of, the amino acid sequence selected from SEQ ID NO: 4, SEQ ID NO: 5, SEQ ID NO: 6 or SEQ ID NO: 7.

More specifically, the targeting element may be covalently linked, directly or through a linker, to the peptide of the invention.

So, in a preferred embodiment, the targeting element is covalently linked, directly or through a linker, to the N-terminal end, C-terminal end, or any other amino (-NH₂), carboxy (-COOH), sulphide (S²-) or reactive groups of the peptide of the invention.

In the present invention, the expression "directly linked" means that the peptide of the invention and the targeting element are synthesised together in a synthesis event (transcription and translation processes) in a selected host organism, such as *Escherichia coli* or, alternatively, they are linked through a spontaneous or chemical reaction involving peptide of the invention and targeting elements.

The expressions "indirectly linked" or "linked through a linker" mean that peptide of the invention and the targeting element are bound through a linker molecule functioning as bridge between the peptide of the invention and the targeting element. Therefore, when the peptide of the invention and the targeting element are linked through a linker, they are not contiguously linked, meaning that the targeting element is linked to the linker and the linker is linked to the N-terminal end, C-terminal end, or any other amino (-NH₂), carboxy (-COOH), sulphide (S²-) or reactive groups of the peptide of the invention.

As used herein, the term "linker" refers to a chemical entity composed of at least one atom (such as carbon, oxygen, nitrogen, sulphur, phosphorous and combinations thereof), that links at least two other entities together. In the case of the invention, the two entities linked through a linker are the peptide of the invention and a targeting element. The linker may be selected from peptidyl linkers and non-peptidyl linkers.

The "peptidyl linker" refers to a peptide that consists of 2 to 50 amino acids in length, preferably 2 to 30 amino acids, more preferably 2 to 20 amino acids, more preferably 2 to 10 amino acids. Peptide linkers may be flexible or rigid linkers depending on their amino acid composition. Peptide flexible linkers comprise small, non-polar (e.g. Gly) or polar (e.g. Ser or Thr) amino acids, while rigid peptides exhibit relatively stiff structures by adopting α-helical conformations or by containing multiple proline residues.

The term "non-peptidyl linker" refers to a linker based on straight or branched chain hydrocarbons or polyethylene glycols of varying lengths. These may incorporate other groups to effect solubility, rigidity, isoelectric point, such as aromatic or non-aromatic rings, halogens, ketones, aldehydes, esters, sulfonyls, phosphate groups, and so on. Examples of non-peptidyl linkers include, without limitation, sulfosuccinimidyl 4-(N-maleimidomethyl)cyclohexane-1-carboxylate, 4-(N-maleimidomethyl)cyclohexane-1-carboxylic 3-sulfo-n-hydroxysuccinimide ester or succinimidyl-4-(N-maleimidomethyl)cyclohexane-1 -carboxy-(3-AmidoCaproate).

Further, the invention refers to a nucleotide sequence, hereinafter "the nucleotide sequence of the invention", coding for the peptide of the invention. As used herein, the term "nucleotide sequence" refers to a polymeric form of any length, either ribonucleotides or deoxyribonucleotides. This term only refers to the primary structure of the molecule. Thus, this term includes DNA from double or single stranded, as well as double or single stranded RNA. It also includes all types of known alterations (e.g. methylation, capping, substitution etc) as well as unmodified forms of the nucleotide sequence.

In a further aspect the invention refers to a "vector" or "expression vector" comprising the nucleotide sequence of the invention, hereinafter the "vector of the invention". The term "vector" herein means the vehicle by which a DNA or RNA sequence of a foreign gene can be introduced into a host cell so as to transform it and promote expression of the introduced sequence. Vectors may include for example, plasmids, phages, and viruses and are discussed in greater detail below. Indeed, any type of plasmid, cosmid, YAC or viral vector may be used to prepare a recombinant nucleic acid construct which can be introduced to a host cell where expression of the protein of interest is desired. Alternatively, wherein expression of the protein of interest in a particular type of host cell is desired, viral vectors that selectively infect the desired cell type or tissue type can be used.

In a further aspect, the invention refers to a host cell comprising the nucleotide sequence of the invention or the expression vector of the invention.

In the context of the present invention, the term "host cell" includes any type of cell that is susceptible to transformation, transfection, transduction, and the like with a nucleic acid construct or expression vector comprising a nucleotide or nucleotide sequence that encodes the peptide of the invention. The host cell can be eukaryotic or prokaryotic. Eukaryotic cells include, but are not limited to, yeast, insect cells, mammalian cells, plants or fungi. Prokaryotes include Gram-negative organisms (for example, *Escherichia coli*) or Gram positive (for example, bacteria of the genus *Bacillus*).

As it has been previously exposed, the inventors found that the peptide of the invention can establish non-covalent interactions with other molecules or particles, such as extracellular vesicles or nanoparticles that can be used for carrying substances comprised in said extracellular vesicles or nanoparticles. Therefore, the peptide of the invention can be used to functionalize an extracellular vesicle or nanoparticle, providing a carrier complex.

Thus, in a further aspect, the invention relates to a carrier complex, hereinafter the "carrier complex of the invention", comprising an extracellular vesicle or a nanoparticle non-covalently linked to the peptide of the invention.

The terms "non-covalently", "non-covalent" or "non-covalent interaction", used interchangeably, refer to chemical interactions that, unlike covalent bonds, do not involve sharing of electrons between atoms. Non-covalent interactions can be ionic interactions or electrostatic interactions, Van der Waals interactions, hydrophobic interactions hydrogen bonds. The peptide of the invention preferably interacts by electrostatic interactions (electrostatically linked) with an EV or nanoparticle. The term "electrostatic interaction" refers to the attractive or repulsive interaction between elements having electric charges.

The term "extracellular vesicles" or "EVs", as used herein, refers to lipid bilayer-delimited particles released from cells, either prokaryotic or eukaryotic cells. The lipid bilayer delimits a core. The lipid bilayer may comprise embedded proteins and other elements such as carbohydrates. The core is a hydrophilic environment that can comprise proteins, DNA and/or RNA. They have an essential role in the intercellular communication since EVs transport cargo such as DNA, RNA and/or proteins between cells. In preferred embodiments of the carrier complex of the invention, the EV is an exosome or a microvesicle. The EV may be selected from a prokaryotic or eukaryotic EV, preferably a prokaryotic EV. The eukaryotic EVs are produced by a cell or organism belonging the any of the eukaryotic kingdoms Animalia, Plantae and Fungi. The prokaryotic EV is produced by cell or organism belonging to the kingdom Monera. In a preferred embodiment, the EV of the carrier complex of the invention is a prokaryotic EV, preferably a prokaryotic EVs of a bacterium belonging to a genus selected from, but not limited to, *Listeria, Lactobacillus, Bacillus, Listeria* or *Bifidobacterium,* preferably *Lactobacillus.*

The term "lipid bilayer" refers to a structure comprising two monolayers containing amphipathic lipid molecules oppositely oriented. Amphipathic lipids comprise a polar headgroup (hydrophilic headgroup) covalently linked to a one or two non-polar (hydrophobic) acyl chains.

The term "exosome" refers to a type of extracellular vesicle characterized by having a particle size ranging from 30 to 250 nm.

The term "microvesicle" refers to a type of extracellular vesicles characterized by having a particle size ranging from 100nm to 1 µm.

EVs have generally negative surface charges, therefore it is a feature affecting the surface chemistry that can be exploited for interaction purposes with the peptide of the invention.

EVs can be isolated by means of already known methods for the skilled person such as those based on selective capture of EVs that bear specific marker proteins on their surface, such as immunoaffinity isolation; or those methods that isolate EVs based on their size such as filtration, size exclusion chromatography or ultracentrifugation. EVs can be loaded with a bioactive agent by using already known methods for the skilled person such as electroporation, direct mixing saponin mediated permeabilization, sonication, freeze-thaw cycles, and extrusion.

The term "nanoparticle" refers to a particle defined as a particle having a size within the nanometre scale, such as for example liposomes, micelles, silica nanoparticles o peptide nanoparticles.

In some preferred embodiments of the carrier complex of the invention, the nanoparticle is selected from a liposome, a micelle, a silica nanoparticle or a peptide nanoparticle.

The term, "liposome" as used herein, refers to a self-assembly spherical structure comprising at least one lipid bilayer. Energetically unfavourable contacts between the hydrophobic acyl chains and the surrounding aqueous medium induce the amphipathic lipid molecules to arrange themselves such that their polar (hydrophilic) headgroups are oriented towards the bilayer's surface, while the acyl chains (non-polar or hydrophobic groups) reorient towards the interior of bilayer. Liposomes are composed of a variety of lipid obtained from natural or synthetic sources. Suitable liposome lipids include, without limitation, phospholipids such as phosphatidylcholines, phosphatidylethanolamines, phosphatidylserines, phosphatidylglycerols, phosphatidylinositols and phosphatidic acids. Liposomes can have a single lipid bilayer (unilamellar liposomes) or multiple lipid bilayers (multilamellar liposomes), wherein each bilayer surrounds, or encapsulates an aqueous compartment. Liposomes may have a variety of sizes ranging from 25nm to 10 µm depending on the lipid composition and method of preparation. Liposomes can be prepared following already known methods for the skilled person (Akbarzadeh, A., et al., (2013). Nanoscale research letters, 8(1), 1-9). The surface charge of the liposomes can be specifically designed using, for instance phosphatidylcholine (to add positive charges) or cholesterol (for negative charges) (Villasmil-Sanchez et al, 2010. DrugDevel Ind Pharm, 36(6), 666-67), or by more elaborate procedure replacing 1,2-dioleoyl-sn-glycero-3-phosphocholine (DOCP) for 1,2-dioleoyl-sn-glycero-3-phosphate (sodium salt) (DOPA), and then obtaining different degrees of negatively charged liposomes, with 1,2-dioleoyl-sn-glycero-3-phospho-L-serine (sodium salt)(DOPS), 1,2-dioleoyl-sn-glycero-3-phospho-(1'-rac-glycerol) (sodium salt) (DOPG) or dicetyl phosphate (DCP), as described (Yanasarn N, Sloat BR, Cui Z. 2011. Mol Pharm. 8, 4, 1174-1185).

The term "micelle" refers to monolayer nanoparticles having a size ranging from 10 to 100 nm, made of amphiphilic polymers with hydrophilic and hydrophobic units. Depending on the hydrophobic and hydrophilic units of the polymer and solvent conditions, the morphologies of micelles take different shapes. Micelles generally have spherical shape in polar solvents, wherein the polar or hydrophilic units of amphiphilic polymers are in the surface of the micelle, while the hydrophobic units are inside the micelle, wherein the hydrophobic units form a hydrophobic core. In non-polar solvents the polymers arrange in an inverse conformation wherein the hydrophobic units of amphiphilic polymers are in the surface of the micelle while the hydrophilic units are inside the micelle, wherein the hydrophilic units form a hydrophilic core. Micelles can be prepared by already known methods for the skilled person such as simple dissolution, dialysis, oil in water emulsion solvent evaporation and lyophilization or freeze drying.

The term "silica nanoparticle" refers in the present invention to inorganic particles composed of silicon oxide or silica (SiO2) and having a size within the range between 1 and 5000 nm, preferably the size is within the range between 1 to 1000nm, more preferably the size is between 100 to 500nm. The silica nanoparticle preferably has spherical morphology, wherein the size refers to the diameter. The silica nanoparticle of the present invention is preferably a mesoporous silica nanoparticle. The term "mesoporous silica nanoparticle" refers to a silica nanoparticle containing pores with diameters between 2 and 50nm. Mesoporous silica nanoparticles can be obtained already known methods in the art (Narayan, R. et al., (2018). Pharmaceutics, 10(3), 118).

The term "peptide nanoparticle" refers to particles that comprise peptides or proteins that arrange in the space to form nanostructures such as, without limitation, spheres, nanorods or prisms, having a size within the range between 5 and 5000 nm, preferably the size is within the range between 5 to 1000nm. Peptides or proteins, being part of peptides nanoparticles my vary in amino acid sequence length, from 5 to 500 amino acids. Peptides or proteins being part of peptide nanoparticles may comprise a plurality of negatively charged amino acids with which the peptide of the invention can electrostatically interact (Duro-Castano, A., Conejos-Sánchez, I., & Vicent, M. J. (2014). Polymers, 6(2), 515-551; Melnyk, T., Ðord̅ević, S., Conejos-Sanchez, I., & Vicent, M. J. (2020). *Advanced drug delivery reviews, 160,* 136-169).

EVs and nanoparticles can be loaded with or can comprise a compound of interest such as a bioactive agent. In the context of the present invention, the term "bioactive agent" refers to any compound or mixture of compounds which produces a physiological result or modulates a biological process, e.g., a beneficial or useful result, a toxic result or the highlighting of a characteristic upon contact with a living organism or cell. Bioactive agents are distinguishable from other components of the delivery compositions, such as carriers, diluents, binders, colorants, etc. The bioactive agent may be a substance used in the diagnosis, treatment, or prevention of a disease.

In some preferred embodiments of the carrier complex of the invention, the EV or the nanoparticle comprises at least one bioactive agent. Thereafter, the peptide of the invention linked to a targeting element can direct the EVs or nanoparticle to a target cell or tissue, or to a target virus, leading to a targeted delivery of a bioactive agent comprised in the EV or nanoparticle. The expression "targeted delivery", as used herein, refers to the delivery of a bioactive agent in a manner that its concentration increases in the target virus, cell or tissue in comparison to other non-targeted virus, cell or tissues. The terms "target virus", "target cell" or "target tissue" refer to a cell, tissue, or virus comprising a target molecule to which the targeting element linked to the peptide of the invention is directed. The targeted delivery may allow to avoid high doses of bioactive agent, it could also help enhance efficacy of the bioactive agent; and prevent toxic side effects that are associated with the delivery of a bioactive agent into a non-targeted cell or tissue. Therefore, the carrier complex of the invention may be used in multiple applications, for example, it can be used to deliver an antibacterial agent or an antiviral agent, to kill pathogens (target bacterium or virus) that have reproduced within a complex organism like in the human intestine, or a plant. Alternatively, the complex of the invention may also be in the context of complex ecosystems such as sewage sludge to specifically reduce the population of, for example, a target bacterium, by the delivery of antibacterial agents.

In some embodiments of the carrier complex of the invention, the bioactive agent may be selected from the list consisting of a peptide, a protein, a DNA, a RNA, a drug, a fluorescent agent, a radiolabelled agent, contrast agent, a phytosanitary agent and any combination thereof.

The term "DNA" (deoxyribonucleic acid) refers to single-stranded or double stranded polymers of nucleotide monomers, including 2' deoxyribonucleotides: adenosine, cytosine, thymine and guanosine. The DNA can be genomic DNA (gDNA) or complementary DNA (cDNA), wherein the gDNA comprises coding and non-coding sequences wherein cDNA only contains coding sequences. cDNA is obtained by the reverse transcription of a mRNA sequence.

The term "RNA" (ribonucleic acid) refers to single-stranded or double stranded polymers of nucleotide monomers, including ribonucleotides: adenosine, cytosine, uracil and guanosine. The RNA can be classified into mRNA and non-coding RNA.

The term "mRNA" (messenger RNA), as use in herein, refers to a single stranded molecule of RNA that comprising a nucleotide sequence coding for a protein.

On the contrast, the term "non-coding RNA" (ncRNA), as used herein, refers to a single or double stranded molecule of RNA that comprises a nucleotide sequence that does not code for a protein. ncRNAs are involved in the regulation of different cellular processes such as, translation, splicing or RNA, gene expression or DNA replication. The ncRNA may be selected from, but not limited to, micro-RNA (miRNA), small interfering-RNA (siRNA), long non coding RNA (IncRNA), antisense RNA or guide RNA (gRNA).

The term "antisense RNA", refers to a non-coding RNA that is complementary to a mRNA with which it hybridizes blocking its translation into protein.

The term "small interfering RNA" (siRNA), refers to a double stranded non-coding RNA having a sequence length ranging from 15 to 30 nucleotides involved in the gene expression regulation of other genes, mainly by its association to the 3'-UTR region of a target mRNA blocking its translation or promoting its degradation. siRNAs regulate the expression of a target mRNA.

The term "micro RNA" (miRNA), refers to a single stranded small non-coding RNA having a sequence length ranging from 15 to 30 nucleotides involved in the gene expression regulation of other genes, mainly by its association to the 3'-UTR region of a target mRNA blocking its translation or promoting its degradation. miRNAs can regulate the expression of multiple mRNAs.

The term "long non coding-RNA" (IncRNA), refers to a non-coding RNA having a having a sequence length of more than 200 nucleotides. LncRNAs participate in the regulation of transcription in different ways such as by the direct interaction with DNA sequences or the interaction with transcription factors.

The term "guide RNA" (gRNA), refers to a non-coding RNA that works as a guide for RNA- or DNA-targeting enzymes, with which it forms complexes, wherein these RNA or DNA- targeting enzymes delete, insert or otherwise alter the targeted RNA or DNA. They occur naturally, serving important functions, but can also be designed to be used for targeted editing, such as with CRISPR-Cas9 and CRISPR-Cas12.

The peptide, protein, DNA and RNA being a bioactive agent may also be referred to as bioactive peptide, bioactive, protein, bioactive DNA and bioactive RNA, respectively.

The term "drug" refers to a molecule having a low molecular weight (≤ 1000 daltons) that can regulate a biological process. In the present invention, examples of drugs molecules include, without limitation, vasodilators, antihypertensive agents, antiarrhythmic agents, hypotensive agents, antitussive agents, local anesthetics, hormones, antiasthmatic agents, antiallergenic agents, antihistamines, anticoagulants, antispasmodics, metabolism modulators, antidepressants, antianxiety agents, vitamins, hypoglycemic agents, immunosuppressant agents, sedative agents, angiogenic agents, antiangiogenic agents, neurotransmitters, psychoactive drugs, anticancer agents, antibacterial agents, antifungal agent, antiviral agents and combinations thereof.

The terms "fluorescent agent", "fluorescent compound", "fluorophore" or "fluorochrome", used interchangeably, refer to that can re-emit light upon light excitation. Fluorophores typically contain several combined aromatic groups, or planar or cyclic molecules with several π bonds. Fluorophores are notably used to stain tissues, cells, or materials in a variety of analytical methods, e.g. fluorescent imaging and spectroscopy. Examples of fluorophores include, without limitation: fluorescent proteins, (e.g. green fluorescent protein), octadecyl rhodamine B, 7-nitro-2-1,3-benzoxadi azol-4-yl, 4- acetamido-4'-isothiocyanatostilbene-2,2' disulfonic acid, acridine and derivatives, 5- (2'-aminoethyl)aminonaphthalene-I-sulfonic acid (EDANS), 4-Amino-N-(3-[vinylsulfonyl]phenyl)naphthalimide-3,6-disulfonate dilithium salt, N-(4-anilino-I-naphthyl)maleimide, anthranilamide, BODIPY, Brilliant Yellow, coumarin and derivatives, cyanine dyes, cyanosine, 4',6-diaminidino-2-phenylindole (DAPI), Bromopyrogallol Red, 7-diethylamino-3-(4'-isothiocyanatophenyl)-4-methylcoumarin, diethylenetriamine pentaacetate, 4,4'-diisothiocyanatodihydro-stilbene-2,2'- disulfonic acid, 4,4'-diisothiocyanatostilbene-2,2'-disulfonic acid, dansylchloride, 4-dimethylaminophenylazophenyl-4'-isothiocyanate (DABITC), eosin and derivatives, erythrosin and derivatives, ethidium, fluorescein, 5-carboxyfluorescein (FAM), 5-(4,6-dichlorotriazin-2-yl)aminofluorescein (DTAF), 2',7'-dimethoxy-4'5'-dichloro-6-carboxyfluorescein, fluorescein isothiocyanate, X-rhodamine-5-(and 6)- isothiocyanate (QFITC or XRITC), fluorescamine, ten-l-yl]ethenyl]-l,l-dimethyl-3-(3- sulfopropyl)-,hydroxide, innersalt compound with n,n-diethylethanamine(I:I) (IR144), 5-chloro-2-[2-[3-[(5-chloro-3-ethyl-2(3H)-benzothiazol-ylidene)ethylidene]-2- (diphenylamino)-l-cyclopenten-l-yl]ethenyl]-3-ethyl benzothiazolium perchlorate (IR140), Malachite Green isothiocyanate, 4-methylumbelliferone, ortho cresolphthalein, nitrotyrosine, pararosaniline, Phenol Red, B-phycoerythrin, o- phthaldialdehyde, pyrene, pyrene butyrate, succinimidyl 1-pyrene, butyrate quantum dots, Reactive Red 4 (Cibacron^{™} Brilliant Red 3B-A), rhodamine and derivatives, 6- carboxy-X-rhodamine (ROX), 6-carboxyrhodamine (R6G), lissamine rhodamine B sulfonyl chloride rhodamine (Rhod), rhodamine B, rhodamine 123, rhodamine X isothiocyanate, sulforhodamine B, sulforhodamine 101, sulfonyl chloride derivative of sulforhodamine 101 (Texas Red), N,N,N',N'tetramethyl-6-carboxyrhodamine (TAMRA) tetramethyl rhodamine, tetramethyl rhodamine isothiocyanate (TRITC), riboflavin, rosolic acid, terbium chelate derivatives, Cyanine-3 (Cy3), Cyanine-5 (Cy5), Cyanine- 5.5 (Cy5.5), Cyanine-7 (Cy7), IRD 700, IRD 800, Alexa 647, La Jolta Blue, phthalo cyanine or naphthalo cyanine. The aforementioned compounds me be present alone or bound, preferably covalently bound, to other molecules such as a proteins or nucleic acids. Another example of fluorescent compounds are fluorescent proteins such as green fluorescent protein (GFP).

The term "radiolabelled agent" or "radiolabelled compound", used interchangeably, refers to a compound, such as a small molecule, a protein, peptide a lipid, a nucleic acid or carbohydrate, comprising radioactive isotopes in its structure. Usually an atom or group of atoms have been replaced by their corresponding isotopes (label). Some examples of radiolabels or isotopes are, without limitation: ¹¹C, ⁶⁴Cu, ¹⁸F, ⁶⁷Ga, ⁶⁸Ga, ¹¹¹In, ¹²³I, ¹²⁵I, ¹³¹I, ¹⁷⁷Lu, ¹³N, ²²³Ra, ¹⁵³Sm, ^{99m}Tc, ²⁰¹TI, ¹³³Xe, and ⁹⁰Y.

The term "contrast agent" is a substance or compound used to increase the contrast or enhance the visibility of a target cell, tissue, structure or organ, wherein said increase in contrast or visibility can be detected by techniques such as X-ray , computed tomography (CT), MR imaging, ultrasound, positron emission tomography (PET), and single photon computerized tomography (SPECT). Examples of contrast agents include, without limitation, a ionidated compound (e.g. I, ioxoglate, iotrolan, ioversol, iothalamate, iodimide, iodipamide,iopromide or metrizamide) barium based compounds( e.g. barium sulphate) or galodinium based compounds (galodinium oxide).

The terms "phytosanitary agent" "phytosanitary product" or "plant protecting product" refer to a compound for treating and/or protecting plants or plant products against any plant pathogen. The term "plant" refers to any plant without limitation, preferably crop plants. Plant product "plant product" refers to any flower, fruit, vegetable, root, bulb, seed, or other plant part or any manufactured or processed plant or plant part. Examples of phytosanitary agents include, without limitation, insecticides, fungicides, bactericides, acaricides, herbicides or nematicides.

As used in the present invention, the term "plant pathogen" refers to any pathogen organism or pathogen agent such as insects, mites, nematodes, fungi, bacteria, viruses or protozoa, that produce a disease or damage to a plant. The plant pathogens of great relevance where this invention could be implemented through the delivery of targeted antibacterial compounds are for instance: *Pseudomonas syringae pathovars, Ralstonia solanacearum, Agrobacterium tumefaciens, Xanthomonas oryzae pv. Oryzae, Xanthomonas campestris pathovars, Xanthomonas axonopodis pathovars, Erwinia amylovora, Dickeya* (dadantii and solani), *Pectobacterium carotovorum,* and *Xylella fastidiosa.*

In a further aspect the invention relates to a composition, hereinafter the "composition of the invention", comprising the peptide of the invention, the polynucleotide of the invention, the vector of the invention, the host cell of the invention or the carrier complex of the invention.

In a preferred embodiment the composition of the invention is a pharmaceutical composition, hereinafter the "pharmaceutical composition of the invention" or a phytosanitary composition, hereinafter the "phytosanitary composition of the invention".

As used in the present invention, the term "pharmaceutical composition" refers to a composition suitable for being administered to a subject for the purpose of restoring, correcting or modifying physiological functions by exerting a pharmacological, immunological or metabolic action, or to establish a medical diagnosis.

In a preferred embodiment, the pharmaceutical composition of the invention comprises the carrier complex of the invention comprising a bioactive agent selected from a peptide, a protein, a DNA, a RNA, a drug, a fluorescent agent, a radiolabelled agent, contrast agent and any combination thereof.

The pharmaceutical composition of the invention can be presented in any clinically permitted form of administration to a subject and in a therapeutically effective amount. For example, it may be in a form adapted for oral, sublingual, nasal, intrathecal, bronchial, lymphatic, rectal, transdermal, intravenous, intraperitoneal, orogastric, intracolonic, inhaled, or parenteral administration. More specifically the pharmaceutical composition can be formulated in the form of a liquid or solid composition.

In the context of the present invention, the terms "subject", "patient" or "individual" are used herein interchangeably to refer to any member of the animal kingdom and can be a vertebrate such as, a fish, a bird, a reptile, an amphibian or a mammal, including a human beings, non-human primate, horse, pig, rabbit, dog, sheep, goat, cow, bird, cat, guinea pig or rodent. In a more preferred embodiment, the subject is a mammal, more preferably a human.

As used in the present invention, the term "phytosanitary composition" refers to a composition intended for protecting plants and/or plant products against plant pathogens.

In a preferred embodiment, the phytosanitary composition of the invention comprises the carrier complex of the invention comprising as a bioactive agent a phytosanitary agent.

The phytosanitary composition can be in the form of liquid or solid state such as wettable powders, oily dispersions, encapsulated granules, capsule suspensions, emulsifiable concentrates or granules, granules, microgranules, microemulsions or water-dispersible granules.

In some preferred embodiments, the pharmaceutical composition further comprises a pharmaceutically acceptable excipient and/or carrier. In some preferred embodiments, the phytosanitary composition further comprises an agriculturally acceptable excipient and/or carrier.

The term "agriculturally acceptable" refers the suitability of a substance or combination of substances which can be used in the agricultural sector.

The term "pharmaceutically acceptable" refers to suitability of a substance or combination of substances to be administered to a subject without causing any harm and allowing the activity of the substances of said composition.

The term "excipient" refers to a substance that aids in the absorption of any of the components of the composition of the present invention, stabilizes said components, or aids in the preparation of the composition Thus, the excipients could have the function of keeping the components together, such as starches, sugars or celluloses; sweeten function; coloring function; protective function of the drug, such as to isolate it from air and/or moisture; function filler of a tablet, capsule, pill or any other form of presentation such as, for example, dibasic calcium phosphate; disintegrating function to facilitate the removal of components; without eliminating other types of excipients not mentioned in this paragraph.

The "vehicle" or "carrier" is preferably an inert substance. Carrier functions are to facilitate the incorporation of other components or compounds, allow better dosage and administration and/or give consistency and form to composition. Therefore, the carrier is a substance used in the drug to dilute any of the components or compounds of the composition of the present invention to a given volume or weight; or that even without diluting these components or compounds, it is able to allow better dosage and administration and/or give consistency. The carrier can be natural or unnatural. Examples of carriers include, without being limited thereto, water, salt solutions, alcohol, vegetable oils, polyethylene glycols, gelatine, lactose, starch, amylose, magnesium stearate, talc, surfactants, silicic acid, viscous paraffin, perfume oil, monoglycerides and diglycerides of fatty acids, fatty acid esters petroetrals, hydroxymethylcellulose, polyvinylpyrrolidone and the like.

In a further aspect, the invention refers to the use of the peptide of the invention or the carrier complex of the invention for the manufacture of a medicament.

In another aspect, the invention refers to the carrier complex of the invention or the pharmaceutical composition of the invention for use as a medicament, wherein the carrier complex of the invention or the pharmaceutical composition of the invention comprise a bioactive agent selected from a peptide, a protein, a DNA, a RNA, a drug, a fluorescent agent, a radiolabelled agent, contrast agent and any combination thereof.

The term "medicament" as used herein means any substance or composition used for the prevention, treatment or diagnosis of a disease in a subject or that may be administered to the subject in order to restore, correct or modify its physiological functions by exerting a pharmacological, immunological or metabolic action.

The terms "treat," "treating" or "treatment," and other grammatical equivalents as used throughout the present description, include alleviating, inhibiting or reducing symptoms, reducing or inhibiting severity of, reducing incidence of, prophylactic treatment of, reducing or inhibiting recurrence of, preventing, delaying onset of, delaying recurrence of, abating or ameliorating a disease or condition symptoms, ameliorating the underlying metabolic causes of symptoms, inhibiting the disease or condition, e.g., arresting the development of the disease or condition, relieving the disease or condition, causing regression of the disease or condition, relieving a condition caused by the disease or condition, or stopping the symptoms of the disease or condition.

The terms "prevent," "preventing" or "prevention," and other grammatical equivalents as used throughout the present description, include preventing additional symptoms, preventing the underlying metabolic causes of symptoms, inhibiting the disease or condition, e.g., arresting the development of the disease or condition and are intended to include prophylaxis. The terms further include achieving a prophylactic benefit. For prophylactic benefit, the compositions are optionally administered to an individual at risk of developing a particular disease, to an individual reporting one or more of the physiological symptoms of a disease, or to an individual at risk of reoccurrence of the disease.

The terms "diagnosis" or "diagnosing", as used herein, refers to a method through which a disease or pathological condition is identified. The terms "monitor" or "monitoring", as used herein, refer to the follow up of the outcome of a disease.

In some preferred embodiments, the invention refers to the carrier complex of the invention or the pharmaceutical composition of the invention for use in the treatment and/or prevention of a disease selected from cancer, a disease caused by a pathogen, an immune disease, cardiovascular disease, metabolic disease, alcoholic liver disease, non-alcoholic liver disease, organ degenerative diseases and a neurological diseases. The use as a medicament or the use for the treatment or the prevention of the aforementioned diseases are directed to a subject, wherein the term subject has been defined in previous aspects being said definition herein applicable as well.

The term "cancer" refers to a pathological condition or disease caused by the uncontrolled growth and spreading of cells. Cancer may be selected from, but not limited to, prostate cancer, colon cancer, breast cancer; gastrointestinal tract cancers, such as gastric cancer, hepatocellular carcinoma, colorectal cancer, pancreatic cancer, gastric cancer, nasopharyngeal cancer, esophageal cancer, cholangiocarcinoma, gall bladder cancer and anogenital cancer; intergumentary cancer, such as skin carcinoma; respiratory tract cancers, such as bronchial cancer and mesothelioma; genitourinary tract cancer and bladder cancer; and reproductive system cancer, such as ovarian cancer.

The term "pathogen, viral or infectious agent", as used in the present invention, refers to any virus, bacterium, protozoan, or fungus which may colonize and cause a disease in a subject.

The pathogenic bacteria may be a Gram positive or Gram negative pathogenic bacteria. The term "Gram-positive bacteria", as used throughout the present description, refers to Gram-positive bacteria which are known and/or can be identified by the presence of certain cell wall and/or cell membrane characteristics and/or by staining with Gram staining. Gram positive pathogenic bacteria are known and can readily be identified and may be selected from, but are not limited to, bacteria belonging to the genera *Listeria, Staphylococcus, Streptococcus, Enterococcus, Mycobacterium, Corynebacterium,* and *Clostridium,* and include all recognized or unrecognized species or strains thereof. The term "Gram-negative bacteria" generally refers to bacteria which produce a crystal violet stain that is decolorized in Gram staining, i. e. they do not retain crystal violet dye in the Gram staining protocol. Gram negative bacteria are known and can readily be identified and may be selected from, but are not limited to, bacteria belonging to the genera *Pseudomonas, Klebsiella, Enterobacter, Escherichia coli, Citrobacter, Salmonella, Yersinia, Shigella, Proteus, Pseudomonas, Vibrio, Legionella, Haemophilus, Bordetella, Brucella, Campylobacter, Neisseria and Branhamella,* including all recognized or unrecognized species or strains thereof.

The pathogenic fungi may belong to genera *Candida, Cryptococcus, Aspergillus, Coccidioides, Histoplasma or Blastomyces,* but it is no limited to the aforementioned genera.

The pathogenic protozoan may belong to the genera *Entamoeba, Trichomonas, Leishmania, Chilomonas, Giardia, Sarocystys, Nosema* or *Histomonas*

The pathogenic virus may be a SARS coronavirus, Coxsackievirus, Dengue virus, Encephalomyocarditis virus, Epstein-Barr virus, Hepatitis A virus, Hepatitis B virus, Hepatitis C virus, Hepatitis E virus, Herpes virus, rhinovirus, influenza virus or rotavirus.

As the skilled person knows, depending on the disease to be treated and or prevented, the bioactive agent of the carrier complex of the invention or the pharmaceutical composition of the invention, is selected accordingly. For example, if the disease is cancer, the bioactive agent is preferably a drug such as an anticancer agent, wherein examples of anticancer agents include, without limitation: nucleoside analogue (e.g. azacytidine, decitabine or fluorouracil), antifolates (e.g. methotrexate) topoisomerase inhibitors (e.g. irotecan or topotecan), anthracyclines (e.g. doxorubicin or epirubicin), taxanes (e.g. cabazitaxel, docetaxel or paclitaxel), vinca alkaloids (e.g. vinblastine or vincristine), alkylating agents (e.g. bendamustine or busulfan), platinum compounds (e.g. carboplatin, cisplatin or oxaliplatin); monoclonal antibodies (e.g cetuximab, denosumab, ipilimumab, nivolumab or rituximab); tyrosine kinase inhibitors (e.g. afatanib axitinib, bosutinib or erlotinib) or mTOR inhibitors (e.g. everolimus or temsirolimus). In another example, in the case of a disease caused by a pathogen, the bioactive agent is preferably a drug such as antibacterial agents, antifungal agent, antiviral agents.

In another aspect, the present invention refers to the use of the carrier complex of the invention or the phytosanitary composition comprising the carrier complex of the invention for the treatment and/or prevention of a disease or diseases caused by a plant pathogen, wherein the bioactive agent is a phytosanitary agent, wherein the carrier complex of the invention or the phytosanitary composition of the invention comprise a phytosanitary agent as bioactive agent, hereinafter the "phytosanitary use of the invention".

The terms "plant", "plant pathogen" and "phytosanitary agent" has been previously defined being said definition applicable in this aspect.

In a preferred embodiment of the phytosanitary use of the invention, the phytosanitary agent is selected from the list consisting of insecticides, fungicides, bactericides, acaricides, herbicides and nematicides.

In a preferred embodiment of the phytosanitary use of the invention, the plant pathogen is selected from the list that consists of insects, mites, nematodes, fungi, bacteria, viruses and protozoa.

In another preferred embodiment of the phytosanitary use of the invention,the plant pathogen is a bacterium selected form the list consisting of: *Pseudomonas syringae pathovars, Ralstonia solanacearum, Agrobacterium tumefaciens, Xanthomonas oryzae pv. Oryzae, Xanthomonas campestris pathovars, Xanthomonas axonopodis pathovars, Erwinia amylovora, Dickeya* (dadantii and solani) and *Pectobacterium carotovorum,* and *Xylella fastidiosa.*

In a further aspect, the invention refers to the *in vitro* use of the peptide of the invention for the targeted delivery of extracellular vesicles or nanoparticles comprising at least one bioactive agent to a target cell or tissue, wherein the bioactive agent is selected from the list consisting of a peptide, a protein, a DNA, a RNA, a drug, a fluorescent agent, a radiolabelled agent, contrast agent, a phytosanitary agent and any combination thereof.

As it is known for a skilled person in the art, fluorescent agent, radiolabelled agent or a contrast agent as previously defined, can be used in imaging methods that are used in the clinic. Imaging methods allow to observe or determine the presence of specific cells or structures, being this information useful for the diagnosis, treatment or follow up of a disease or pathological condition affecting a human or animal. Imaging methods used in the clinic are for example used for the detection of pathological tissues or cells such as tumoral cells or tumour tissues allowing its diagnosis or to monitor its outcome.

Thus, in a further aspect, the invention refers to the carrier complex of the invention or a composition comprising it for use as an imaging agent "hereinafter the imaging use of the invention", wherein the bioactive agent is a fluorescent agent, radiolabelled agent or a contrast agent. The terms "fluorescent agent", "radiolabelled agent" or "contrast agent" have been defined in previous aspects, being said definitions applicable in this aspect. The imaging use of the invention is a *in vivo* use in a subject.

As used herein, the term "imaging agent" refers to a substance or composition that may be used to highlight a specific area of a subject, rendering organs, blood vessels, tissues, and/or other portions to be more detectable and/or more clearly imaged. By increasing the detectability and/or image quality of the specific area of the subject, the presence and extent of disease and/or injury can be determined. Thus, in the context of the present invention, the carrier complex of the invention comprising a bioactive agent selected from a fluorescent agent, radiolabelled agent, and contrast agent, is considered as an imaging agent. The imaging agent emits a signal that can be detected by imaging techniques that can include but are not limited to, fluorescent imaging, computed tomography (CT), magnetic resonance imaging (MRI), optical imaging, single photon emission computed tomography (SPECT), positron emission tomography (PET), x-ray imaging, gamma ray imaging, and the like.

In another aspect, the invention relates to the *in vitro* use of the carrier complex of the invention for the detection of a virus, a bacterium and/or a cell, in an isolated sample from a subject or a plant, wherein the bioactive agent is a fluorescent agent, a contrast agent or radiolabelled agent.

In a preferred embodiment, the virus, bacterium and/or cell detected in the in vitro use of the carrier complex of the invention are a target virus, a target bacterium and/or a target cell.

The terms "target virus", "target bacterium" and "target cell" refer to a virus, tissue, or cell comprising a target molecule to which the targeting element linked to the peptide of the invention is directed.

The "subject" refers to any member of the animal kingdom and can be a vertebrate such as, a fish, a bird, a reptile, an amphibian or a mammal, including a human beings, non-human primate, horse, pig, rabbit, dog, sheep, goat, cow, bird, cat, guinea pig or rodent. In a more preferred embodiment, the subject is a mammal, more preferably a human.

The sample isolated from the subject refers to any tissue sample or biological liquid sample isolated from the subject, such as but not limited to, blood, plasma, serum, sputum, epithelial tissue, urine or sputum.

The plant refers to any pant, preferably crop plants. The sample isolated from a plant refers to a sample of a tissue selected from, leaf, root, fruit and plant stem.

### DESCRIPTION OF THE DRAWINGS

Figure 1. Sensorgrammes illustrating the affinity of P40 fractions to heparin.
Figure 2. Western blots showing the ability of A) peptide P40N1 and B) peptide P40N2 to bind EVs from different bacteria.
Figure 3. Western blots showing the ability of fusion proteins P40N1-APS and P40N1-LQS to EVs from A) *Lacticaseibacillus paracasei* BL23, B) *Listeria monocytogenes* BL1001, C) *Escherichia coli* BE72 and D) *Bifidobacterium animalis* BL230.
Figure 4. Quantitation of fluorescence signal after incubation Caco2 cells with P40N1-APS and P40N1-LQN. Detection of anti-P40 with anti-rabbit-Alexa Fluor.

### EXAMPLES

### MATERIALS AND METHODS

### Preparation of extracellular vesicles from different bacterial species

Extracellular vesicles from *Lacticaseibacillus paracasei, Bifidobacterium animalis, Escherichia choli* and *Listeria monocytogenes* were prepared as described before in Bäuerl C, et al., Scientific Reports 2020, 10(1):19237. Briefly, 200-400 mL of bacterial strains grown on appropriate broth were centrifuged at 10,000 x g for 20 min at 4°C in 250 ml polypropylene bottles and type 16.250 JLA rotor (Avanti J-26 XPI centrifuge, Beckman Coulter, USA). The supernatant was then filtered through a 0.45 µm membrane (Sarstedt, Germany) and EV were concentrated from the clarified medium by ultracentrifugation (UC) at 100,000 x g for 120 min at 4°C in 26.3 mL polycarbonate bottles in type 70 Ti rotor (Beckman Optima L-70, Beckman Coulter, USA). The supernatant was discarded and the pellet was washed with phosphate buffered saline solution (PBS) and ultracentrifuged again at 100,000 x g for 60 min at 4°C. The supernatant was discarded and the pellet containing EVs was resuspended with sterile PBS and stored at -80°C until further use.

**Cloning, expression and purification of peptides P40N1, P40N2 and fusion proteins containing P40N1 fused to peptides recognizing cancer cells.**

Plasmid pQE80e carrying RGS-His (6xHis) epitope encoding region in the cloning site was used to clone, express, and purify P40N1 (SEQ ID NO: 1), P40N2 (SEQ ID NO: 2) and fusion proteins comprising P40N1 fused to peptides recognizing cancer cells (targeting peptides), following the process as described in the Bäuerl C, et al. Frontiers in Microbiology 2019, 10(1420). The peptides LQNAPRS (SEQ ID NO: 4) APSPMIW (SEQ ID NO: 5) are both peptides recognizing cancer cells (Sun, J., et al., (2012) Clinical & experimental metastasis, 29(3), 185-196), and were used to synthetised fusion proteins in combination with peptide P40N1:

Fusion P40N1-LQN, SEQ ID NO: 9:

DTSDSIASNKSETNALLKQIEDANTEVINLNKQIDTKNGQISDATAKISATDAKISSLSGE
IVAAQKNVTARKNNLKDQLISLQKKAGNSVSGNVYIDFVLNSQNLSDLIARTMTVGKLS
QASKDALDAVTVAKDKLAGLKTEQENARQTLVSTKASLETQKSQLVSLQKDANDKQD
ALNKQLQNAPRS

Sequence SEQ ID NO: 9 corresponds to the combination of SEQ ID NO:1 with SEQ ID NO: 4, wherein SEQ ID NO:4 is linked to the C-terminal end of SEQ ID NO: 1.

Fusion P40N1-APS, SEQ ID NO: 10:

DTSDSIASNKSETNALLKQIEDANTEVINLNKQIDTKNGQISDATAKISATDAKISSLSGE
IVAAQKNVTARKNNLKDQLISLQKKAGNSVSGNVYIDFVLNSQNLSDLIARTMTVGKLS
QASKDALDAVTVAKDKLAGLKTEQENARQTLVSTKASLETQKSQLVSLQKDANDKQD
ALNKQAPSPMIW

Sequence SEQ ID NO: 10 corresponds to the combination of SEQ ID NO:1 with SEQ ID NO: 5, wherein SEQ ID NO:5 is linked to the C-terminal end of SEQ ID NO: 1.

Addition of new DNA fragments at the carboxy-terminal region of P40N1 encoding cancer recognizing peptides LQNAPRS (SEQ ID NO: 4) or APSPMIW (SEQ ID NO: 5) was achieved through the complete amplification of pQE-P40N carrying plasmid with the following primers (sequences are provided in the 5'->3'direction):
Primer forward (Fw) LCP40N_for, SEQ ID NO 11:
   GTTGGATCCGATACAAGCGACAG
Primer reverse (rev) LcaP40N1-rev. SEQ ID NO 12:
   GTAGGGCCCTTATTGCTTATTCAAAGC
Primer reverse LCP40N2-rev, SEQ ID NO 13:
   GTTGGTACCTTAGTTGCCTGAACTGC
CD133_cancer recognizing peptide APSPMIW primer Fw_v2, SEQ ID NO 14:
   GCTCCGTCTCCGATGATCTGGTAACCTTAATTAGCTGAGCTTGG
CD133_ cancer recognizing peptide LQNAPRS primer Fw_v2, SEQ ID NO 15:
   CTGCAGAACGCTCCGCGTTCTTAACCTTAATTAGCTGAGCTTGG

Constructs were initially transformed in *E. coli* DH10B to check the sequence quality and then subcloned in E. coli BL21(BE3)-[pLysS], where expression was induced with IPTG. For protein purification, recombinant bacteria were grown in 500 ml of LB supplemented with 100 µg/ml ampicillin and 20 µg/ml chloramphenicol at 37°C. When bacterial cells reached an OD600 nm of 0.4, 1 mM IPTG was added and growth continued for 3 h. The bacterial pellets were recovered by centrifugation and resuspended in 30 ml of Binding Buffer A-His (50 mM Tris-CI pH 7.5, 100 mM NaCl, 50 mM Na2SO4) containing 0.5 mM PMSF and 0.5 mg/ml lysozyme. Bacteria were lysed by sonication, and cell debris was removed by centrifugation at 10000 × g for 5 min at 4°C. The resulting supernatant was filtered (pore-size 0.45 µm) and loaded onto a HisTrapTM FF Crude Column (GE Healthcare Bio-Sciences AB, Uppsala, Sweden) using an ÅktaPrimeTM Plus chromatography system (GE Healthcare Bio-Sciences AB, Uppsala, Sweden), and Histagged proteins were separated according to the instructions of the manufacturer.

### P40N1, P40N2 and fusion proteins binding to EV assay

Adhesion of P40N1, P40N2 and fusion proteins (P40N1-LQN and P40N1-APS) to extracellular vesicles was monitored by western blot, using anti-P40 to detect P40N1, P40N2 and fusion protein. Western blot was performed as Bäuerl C, et al., Scientific Reports 2020, 10(1):19237, using as primary antibody Anti-P40 obtained by the immunization of rabbits with P40N2 overexpressed and purified from *E.coli* (Bäuerl C, et al., Frontiers in Microbiology 2019, 10(1420)). EVs from *Lacticaseibacillus paracasei, Bifidobacterium animalis, Escherichia coli and Listeria monocytogenes* were used in this assays. Binding of P40N1, P40N2, P40N1-LQN and P40N1-APS to EV of different bacteria was assayed as follows, EV corresponding to 2 µg protein were incubated with 0.5 µg of the appropriate purified protein for 60 minutes at room temperature. Then, excess of P40N1, P40N2, P40N1-LQN and P40N1-APS was eliminated by washing twice with PBS by ultrafiltration through a 100,000 kDa centrifugation units (Amicon 0,5ml 100,000 kDa, Merk Millipore, Tullagreen, Ireland). Remaining EV were brought to a volume of 30 µl, from which 15 µl were mixed 5x loading buffer, denatured for 10 at 95°C and then loaded onto a polyacrylamide gel to run an electrophoresis and start the western blot procedure.

### Surface plasmon resonance (SPR) analysis

Binding of P40N1 (SEQ ID NO:1), P40N2 (SEQ ID NO:2) and P40T (SEQ ID NO:3)to heparin was determined by SPR using a Biacore T100 instrument (Biacore, GE Healthcare). Heparin was immobilized on the surface of CM5 chips (GE Healthcare) with the Amine Coupling Kit (GE Healthcare) following the manufacturer's instructions. Heparin was immobilized at 4400-5900 resonance units (RU) in channel 2 and 4 of the CM5 chips and the channels 1 and 3 were left as references. Several fold dilutions of each of the proteins were used as indicated in the graphs and they were injected in the CM5 chip at a fow rate of 30 µl/min in 100 mM HBS-Ep buffer (GE Healthcare) at 25 °C. Kinetic parameters were determined by the Single Cycle procedure at a fow rate of 10 µl/min and constant evaluations obtained with the Biacore Evaluation Sofware.

### Quantitative Immunofluorescence assay

Adhesion of peptide P40N1 and fusion proteins P40N1-LQN and P40N1-APS to the surface of adenocarcinoma cell line (Caco-2) was determined by immune-chemistry followed by fluorescent spectroscopy. Caco2 colon adenocarcinoma cells were maintained in DMEM, 10% SFB and 1% penicillin/streptomycin (Gibco^{™}). Caco2 medium was supplemented with 1% non essential amino acids (Gibco^{™}), 1% sodium pyruvate (Gibco^{™}), 1% HEPES (Gibco^{™}) and 1% L-glutamine 2 mM (Gibco^{™}). For adhesion assays, Caco2 cells inoculated to 96-well black plates at a density 5×10⁴ cells per well, and incubated for 48 h. The cells were washed with 1X PBS and subsequently, incubated for 1 h with 0,50 µg of the different P40N1 domain and fusion proteins P40N1-LQN and P40N1-APS in DMEM medium without antibiotics or supplements at 37 °C. Untreated cells controls were always included. Cells were washed 3 times with PBS and fixed with 100 µL 4% PFA for 10 min at room temperature under dark conditions. Then, cells were washed 3 times again and incubated with 100 µL of blocking solution (4% BSA in PBS) for 1 h at room temperature. After blocking, cells were incubated with 100 µL of the rabbit anti-P40N primary antibody (Sigma Aldrich) at a 1: 400 ratio in 1% BSA in PBS. Subsequently, the cells were washed 3 times with PBS and incubated with 100 µL of the Alexa Fluor 594 conjugated anti-rabbit secondary antibody (Thermofisher Scientific) in a ratio of 1: 500 in 1% BSA in PBS for 1 h at room temperature. Cells were washed 3 times with PBS and 20 µL of PBS was added to perform the measurement. Fluorescence was measure at 540/610 nm using the ClarioStar microplate reader (BMG LABTECH).

### Microscopic observation of adhesion

For the observation of adherence of P40N1, P40N1-APS and P40N1-LQN cell cultures were incubated with peptides for 10 min at 37 °C and 5% CO2. Caco2 cells were seeded on glass coverslips at densities of 3x105, and they were incubated for 24 or 48 h, until >80% confluence. Cells were washed with PBS and treated with P40N1, P40N1-APS and P40N1-LQN in medium without SFB and antibiotics, for 10 min at 37 °C and 5% CO2, untreated cells were used as control. Cells were fixed with 4% paraformaldehyde for 10 min in the dark. Then, were washed and blocked with 4% BSA (Sigma-Aldrich) during 1 h. To detect P40N1, P40N1-APS and P40N1-LQN recombinant peptides primary anti-P40N antibody was used in a ratio of 1:400 in 1% BSA, incubated 1 h. Also, the secondary antibody anti-rabbit Alexa Fluor 488 (Invitrogen) was used in a ratio of 1:500 in 1% BSA. Nuclei were stained with DAPI (Sigma-Aldrich) 1:200 during 10 min in the dark. Samples were mounted in slides using ProLong Diamont Antifade Mountant (ThermoFisher Scientific) and then, visualized by Fluorescence Microscopy (Nikon Eclipse E90i, Nikon Corporation, Japan). Images were captured and processed using the software Nis Elements BR 3.2 (Nikon Corporation, Japan) and the filters DAPI 340-380/435-475 (blue), FITC 465-495/515-555 (green) and G-2a 510-560/565-615 (red).

### RESULTS

### Example 1 Binding of P40N1 and P40N2 to a negatively charged molecule

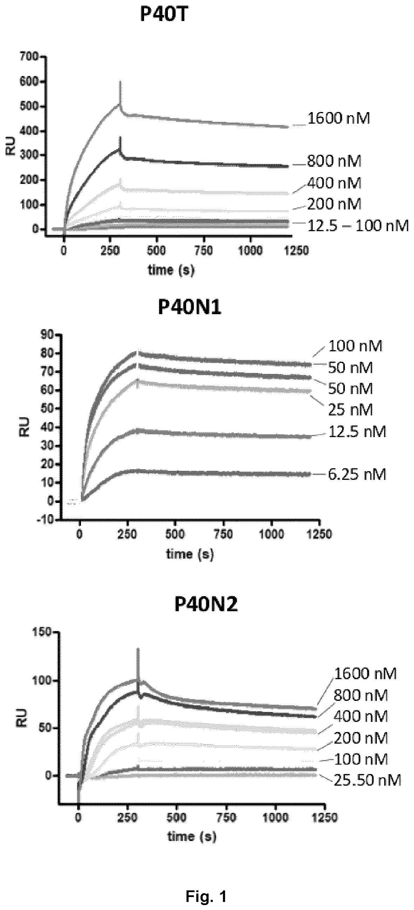

The inventors tested the affinity of peptides P40N1 SEQ ID NO: 1 and P40N2 SEQ ID NO: 2 for negatively charged macromolecules like heparin. The binding kinetics of the interactions between P40N1 and P40N2 with heparin are shown in table 1. The figure 1 shows the sensorgrammes illustrating the affinity of the fractions to heparin.

**Table 1. Binding kinetics of P40 subdomains or fragments to heparin**

| Protein | Sequence ID | Ka (1/Ms) | Kd (1/s) | KD (M) |
|---|---|---|---|---|
| P40T | SEQID NO: 3 | 2188.74 | 1.4 × 10 ⁻⁴ | 6.37 × 10⁻⁸ |
| P40N2 | SEQ ID NO :2 | 234632.58 | 7.11 × 10 ⁻⁵ | 3.03 × 10⁻¹⁰ |
| P40N1 | SEQ ID NO: 1 | 8642.67 | 3.43 × 10 ⁻⁴ | 3.97 × 10 ⁻⁸ |

### Example 2 Binding of P40N1 and P40N2 to EVs from different bacteria

Purified positively charged peptides P40N1 SEQ ID NO: 1 and P40N2 SEQ ID NO:2 have great affinity for bacterial EV, possibly due to the great affinity for the negative charges of lipoteichoic acid. In fact, P40N1 can displace native P40 from the surface of *L. paracasei* EV, efficiently bind to *Listeria monocytogenes* EV and bind to *Eschericia coli* EV possibly competing with native perivesicular proteins (Figure 2A). P40N2 also binds to EV of *L. paracasei, Bacillus subtilis* and *L. monocytogenes* (Figure 2B).

### Example 3 Binding of fusion proteins containing P40N1 fused to cancer recognizing peptides, to EVs from different bacteria.

The newly developed recombinant peptides have been tested on different bacterial EVs. P40N1-APS and P40N1-LQN could bind to EVs from *Lacticaseibacillus paracasei* (Figure 3A), *Listeria monocytogenes* (Figure 3B), *Escherichia coli* (Figure 3C) and *Bifidobacterium animalis* (Figure 3D) likely due to the interaction of the portion P40N1 with negatively charged molecules of the surface of EVs like lipoteichoic acid.

### Example 4 Binding of P40N1-APS and P40N1-LQN to adenocarcinoma cells (Caco-2)

Antibody detection of the P40N1 fraction of the new fusion proteins P40N1-APS and P40N1-LQN could be detected by the increase in fluorescent signal under epifluorescence microscope. A quantitative method based on immune detection of P40N1 domains was adapted to measure the adhesion of proteins to the surface of Caco-2 cells. Results revealed that addition of both heptapeptides (APSPMIW and LQNAPRS) to the sequence of P40N1 multiplied by a factor of 5 the adhesion capability of P40N1 to the surface of Caco-2 cells, adenocarcinoma cells (Figure 4).

## Claims

1. A peptide comprising an amino acid sequence with at least 80% sequence identity to the amino acid sequence SEQ ID NO: 1, wherein the length of the peptide is less than 300 amino acids.

2. The peptide according to claim 1, wherein at least 1 out of 10 amino acids of the amino acid sequence is a positively charged amino acid.

3. The peptide according to any one of claims 1 or 2, comprising the amino acid sequence SEQ ID NO: 1 or SEQ ID NO: 2, preferably consisting of the amino acid sequence SEQ ID NO: 1 or SEQ ID NO: 2.

4. The peptide according to any one of claims 1 to 3, further comprises a targeting element covalently linked to the N-terminal end, C-terminal end, or any other amino (-NH₂), carboxy (-COOH), sulphide (S²-) or reactive groups, of said peptide.

5. The peptide according to claim 4, wherein the targeting element is selected from the list consisting of: a RNA aptamer, a DNA aptamer, a polysaccharide, a targeting peptide, a protein, an antibody and a fragment thereof.

6. The peptide according to claim 5, wherein the targeting peptide comprises the amino acid sequence SEQ ID NO: 4, SEQ ID NO: 5, SEQ ID NO: 6 or SEQ ID NO: 7.

7. A carrier complex comprising an extracellular vesicle (EV), or a nanoparticle, non-covalently linked to the peptide according to any one of the claims 4 to 6.

8. The carrier complex according to claim 7, wherein the nanoparticle is selected from a list consisting of: a liposome, a micelle, a silica nanoparticle and a peptide nanoparticle.

9. The carrier complex according to any one of claims 7 or 8, wherein the extracellular vesicle (EV) or the nanoparticle comprises at least one bioactive agent.

10. The carrier complex according to claim 9, wherein the bioactive agent is selected from the list consisting of: a bioactive peptide, a bioactive protein, a DNA, a RNA, a drug, a fluorescent agent, a radiolabelled agent, a contrast agent, a phytosanitary agent and any combination thereof.

11. A composition comprising the peptide according to any one of claims 1 to 6 or the carrier complex according to any one of claims 7 to 10, preferably wherein the composition is a pharmaceutical composition or a phytosanitary composition.

12. A carrier complex according to any one of claims 9 or 10, or a pharmaceutical composition according to claim 11, for use as medicament.

13. A carrier complex according to any one of claims 9 or 10, or a pharmaceutical composition according to claim 11, for use in the treatment and/or prevention of cancer, a disease caused by a pathogen, an immune disease, cardiovascular disease, metabolic disease, alcoholic liver disease, non-alcoholic liver disease and a neurological disease.

14. Use of the carrier complex according to any one of claims 9 or 10, or the phytosanitary composition according to claim 11, for treatment and/or prevention of diseases caused by a plant pathogen, wherein the bioactive agent is a phytosanitary agent.

15. *In vitro* use of the peptide according to any one of claims 4 to 6 for the targeted delivery of extracellular vesicles or nanoparticles comprising at least one bioactive agent to a target cell or tissue, wherein the bioactive agent is selected from the list consisting of a peptide, a protein, a DNA, a RNA, a drug, a fluorescent agent, a radiolabelled agent, contrast agent, a phytosanitary agent and any combination thereof.

16. A carrier complex according to any one of claims 9 or 10, or a composition comprising it, for use as an imaging agent, wherein the bioactive agent is a fluorescent agent, a contrast agent or radiolabelled agent.

17. *In vitro* use of the carrier complex according to any one of claims 9 or 10, for the detection of a virus, a bacterium and/or a cell, in an isolated sample from a subject or a plant, wherein the bioactive agent is a fluorescent agent, a contrast agent or radiolabelled agent.
